# EUROPEAN PATENT SPECIFICATION

(11) **EP 2 076 482 B1**
(45) Date of publication and mention of the grant of the patent: **11.08.2010**
(21) Application number: 07834772.1
(22) Date of filing: 16.10.2007
(51) Int. Cl.: C07C 51/15, C07C 67/39, C07C 59/19, C07C 69/72

(54) **METHOD FOR THE PRODUCTION OF ALPHA-KETO ACIDS AND ESTERS THEREOF**
VERFAHREN ZUR HERSTELLUNG VON ALPHA-KETO-SÄUREN UND ESTERN DAVON
PROCEDE DE FABRICATION D'ACIDES ALPHA-CETO ET DE LEURS ESTERS

(30) Priority: 17.10.2006 NO 20064703; 18.05.2007 NO 20072536
(43) Date of publication of application: 08.07.2009
(73) Proprietor: GE HEALTHCARE AS, 0401 Oslo (NO)
(72) Inventor: ENGELL, Torgrim, 0401 Oslo (NO); GLØGÅRD, Christian, 0401 Oslo (NO)
(74) Representative: Wulff, Marianne Weiby
(86) International application number: PCT/NO2007/000360
(87) International publication number: WO 2008/048106

(56) References cited:
- WO-A-2006/038811
- JP-A- 8 183 753
- THIRKETTLE J E ET AL: "The origin of the beta-lactam carbons of clavulinic acid" 1997, CHEMICAL COMMUNICATIONS - CHEMCOM, ROYAL SOCIETY OF CHEMISTRY, GB, PAGE(S) 1025-1026 , XP002358982 ISSN: 1359-7345 the whole document
- HOLLSTEIN U ET AL: "SYNTHESIS OF SHIKIMIC-6-13C ACID" 1980, JOURNAL OF LABELLED COMPOUNDS AND RADIOPHARMACEUTICALS, JOHN WILEY, CHICHESTER, GB, PAGE(S) 289-296 , XP008057202 ISSN: 0362-4803 the whole document

## Description

The invention relates to a method for the production of α-keto acids and esters thereof, in particular to a method of producing ¹³C₁-α-keto acids and esters thereof i.e. α-keto acids and esters thereof which are ¹³C-enriched at the C1-atom (carboxyl atom). More particularly, the invention relates to a method of producing pyruvic acid and esters thereof, in particular ¹³C₁-pyruvic acid and esters thereof.

α-Keto acids and derivatives thereof play important roles not only in organic synthesis but also in biologically active natural products. Pyruvic acid and derivatives thereof, especially the salts of pyruvic acid, i.e. pyruvates, are important intermediate found in the pathway of carbohydrate metabolism within the living body. In order to gain insight in this metabolic pathway, ¹³C-isotopically enriched pyruvate can be used to detect metabolites generated in the living body by ¹³C-NMR.

Further, hyperpolarised ¹³C-pyruvate may be used as magnetic resonance (MR) imaging (MRI) agent for *in vivo* MR study of metabolic processes in the human body. The term "hyperpolarised" denotes an enhanced nuclear polarisation of the ¹³C-nuclei present in the pyruvate molecule. Upon enhancing the nuclear polarisation of the ¹³C-nuclei, the population difference between excited and ground nuclear spin states of these nuclei is significantly increased and thereby the MR signal intensity is amplified by a factor of hundred and more. When using a hyperpolarised ¹³C-pyruvate, there will be essentially no interference from background signals as the natural abundance of ¹³C is negligible and thus the image contrast will be advantageously high. Hyperpolarised ¹³C-pyruvate may for instance be used as an MR imaging agent for *in vivo* tumour imaging as described in detail in WO-A-2006/011810 and WO-A-2006/011809 and for assessing the viability of myocardial tissue by MR imaging as described in detail in WO-A-2006/054903. Hyperpolarised ¹³C-pyruvate is produced by for instance dynamic nuclear polarisation (DNP) of either ¹³C-pyruvic acid with subsequent conversion to ¹³C-pyruvate or by directly using ¹³C-pyruvate in the DNP process. The production of hyperpolarised ¹³C-pyruvate is described in detail in WO-A-2006/011809.

In the body, pyruvate is converted (metabolised) into different compounds: its transamination results in alanine, via oxidative decarboxylation, pyruvate is converted into acetyl-CoA and carbon dioxide (which is further converted to bicarbonate), the reduction of pyruvate results in lactate and its carboxylation in oxaloacetate.

Hyperpolarised ¹³C₁-pyruvate, i.e. pyruvate that is isotopically enriched at the C₁-atom (carboxyl atom) is of most interest to be used as MR imaging agent since it has a long T₁ relaxation in human full blood (42 s at 37 ° C), which allows to real-time monitor and detect by MR its conversion to hyperpolarised ¹³C-lactate, hyperpolarised ¹³C-bicarbonate and hyperpolarised ¹³C-alanine.

Several methods for the production of pyruvic acid are known in the art which can be grossly divided into methods involving the use of microorganisms or enzymes and chemical synthesis.

An example of an enzyme based method for the production of pyruvic acid is the enzymatic oxidation of lactic acid, as for instance described in WO-A-95/00656. Enzymatic oxidation often results in byproducts as hydrogen peroxide is produced during said enzymatic oxidation. Further, an upscale of enzymatic processes to an industrial process level is often problematic or impossible. Examples for methods involving the use of microorganisms for the production of pyruvic acid are for instance described in EP-A-313 850. A disadvantage of these microbiological production processes is that it is often difficult and time consuming to separate, isolate and purify pyruvic acid from the complex reaction mixtures, e.g. form complex fermentation broths.

Examples of chemical synthesis for the production of pyruvic acid are largely based on the oxidation of various starting materials like propylene glycol (as described in EP-A-337 246), hydroxyacetone (described in US-A-4 247 716) or lactic acid (see for instance JP-A-8183753). However, for the production of isotopically enriched ¹⁴C- or ¹³C-pyruvic acid, the use of a commercially available isotopically enriched starting material or an isotopically enriched starting material that is obtainable by a straightforward chemical synthesis is greatly preferred.

S. Anker, J. Biol. Chem 176, 1948, 133-1335 describes the synthesis of 2-¹⁴C-enriched pyruvic acid which is obtained by using to synthesizing 1-^{14C}-potassium acetate from ¹⁴C-enriched barium carbonate, converting it to 1-¹⁴C-acetyl bromide and subsequently reaction with cuprous cyanide to 1-¹⁴C-acetyl cyanide. The acetyl cyanide is reacted to 2-^{14C}-pyruvamide which is then hydrolysed to 2-^{14C}-pyruvic acid. Using isotopically enriched cuprous cyanide and acetyl bromide allows the synthesis of a pyruvic acid that is isotopically enriched at the C1-atom; however, the use of toxic cyanides limits the application of this method to low scale synthesis of C1/C2-labelled pyruvic acid.

We have now surprisingly found a method to produce pyruvic acid and pyruvic acid which is ¹³C-enriched at the C1-atom in excellent purity and high yield. Said method is generally useful to produce α-keto acids and esters thereof, in particular α-keto acids and esters thereof which are ¹³C-enriched at the C1-atom

Hence the invention provides a method for producing an α-keto acid of formula (I) or an ester thereof 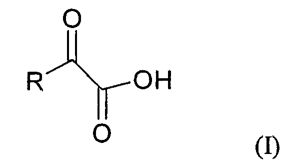 wherein R is a straight chain or branched C₁-C₆-alkyl group or phenyl or benzyl, optionally substituted with one or more hydroxyl or carboxyl groups, said method comprising:
a) obtaining a compound of formula (II) 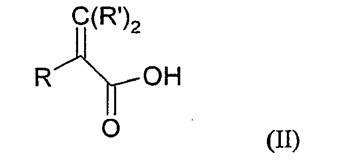 wherein R is defined as above and R' is the same or different and denotes H or straight chain or branched C₁-C₃-alkyl, by carboxylation of a compound of formula (III) 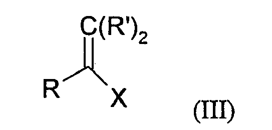 wherein R and R' are as defined above and X is Cl, Br or I;
b) optionally converting the compound of formula (II) into an ester;
c) oxidising the compound of formula (II) or the ester thereof to obtain an α-keto acid of formula (I) or an ester thereof; and
d) if a ester of the α-keto acid of formula (I) was obtained in step c), optionally converting the ester into a α-keto acid.

The term "carboxylation" denotes a chemical reaction in which a carboxylic acid group is introduced into a substrate. In the method of the invention, said substrate is a compound of formula (III).

The α-keto acid of formula (I) may be obtained by the method of the invention in form of the free acid or in the form of a salt. By way of example the method of the invention may be used to produce pyruvic acid and said pyruvic acid may be obtained in form of the free acid or in the form of a salt, i.e. a pyruvate, for instance a sodium pyruvate. Which form is obtained will depend on the work-up procedure of the crude reaction product obtained in step c) and said work-up procedures will be discussed later in the application. Hereinafter and unless indicated otherwise the term "α-keto acid" denotes both the free α-keto acid and salts of the α-keto acid. By way of example, the term "pyruvic acid" thus denotes the free acid and salt of pyruvic acid, i.e. pyruvates.

In the above-mentioned method, R' is the same or different and denotes H or methyl, ethyl, n-propyl or isopropyl. The term "the same or different" means that each of the two R'-groups in formulae (II) and (III) are either different chemical entities or that the two R'-groups in formulae (II) and (III) are identical chemical entities. By way of example, if both R' groups in formula (II) are methyl, R' is the same. If one R' group in formula (II) is H and one R' group in formula (II) is methyl, R' is different.

In a preferred embodiment, R' is the same and denotes H. In another preferred embodiment, R' is different and denotes H and methyl. Embodiments wherein R' is methyl, ethyl, n-propyl or isopropyl may facilitate oxidation in step c) compared to embodiments wherein R' is H.

In a preferred embodiment of the method of the invention, R is methyl, ethyl, n-propyl, isopropyl, n-butyl, isobutyl or tert-butyl. In a further preferred embodiment, R is phenyl or benzyl. In a further preferred embodiment of the invention, R is substituted with one or more hydroxyl or carboxyl groups and is preferably CH₂-COOH, CH₂-CH₂-COOH, CH₂-(CH₂)₂-COOH, CH₂-(CH₂)₃-COOH or CHOH-(CHOH)₂-CH₂OH. In a most preferred embodiment, R is methyl.

In a further preferred embodiment, the method of the invention is used to produce ¹³C₁-α-keto acids or esters thereof, i.e. α-keto acids or esters thereof which are ¹³C-enriched at the C1-atom (carboxyl atom). The ¹³C-enrichment the α-keto acids or esters thereof produced by the method of the invention is preferably at least 75 %, more preferably at least 80 % and especially preferably at least 90 %, an isotopic enrichment of over 90 % being most preferred. Ideally, the enrichment is 100 %.

Preferred α-keto acids or esters thereof - either or not ¹³C₁-enriched - which can be produced according to the method of the invention are phenylglyoxylic acid or esters thereof, phenylpyruvic acid or esters thereof, 2-oxopropanoic acid, i.e. pyruvic acid, or esters thereof, 2-oxobutanoic, i.e. α-keto butyric acid or esters thereof, 2-oxopentanoic acid, i.e. α-keto valeric acid, or esters thereof, 2-oxo-3-methylbutanoic acid, i.e. α-keto isovaleric acid or esters thereof, 2-oxohexanoic acid, i.e. α-keto caproic acid or esters thereof, 2-oxo-4-methylpentanoic acid, i.e. α-keto isocaproic acid, or esters thereof, 2-oxobutanedioic acid, i.e. α-keto succinic acid, or esters thereof, 2-oxopentanedioic acid, i.e. α-keto glutaric acid, or esters thereof, 2-oxohexanedioic acid, i.e. α-keto adipic acid, or esters thereof, 2-oxoheptanedioic acid, i.e. α-keto pimelic acid, or esters thereof. Another preferred α-keto acid or esters thereof which can be produced according to the method of the invention is 2-oxo-3, 4, 5, 6-tetrahydroxyhexanoic acid, i.e. α-keto gluconic acid, or esters thereof.

In a most embodiment, the method of the invention is used to produce pyruvic acid or esters thereof. In a more preferred embodiment, the method of the invention is used to produce ¹³C₁-pyruvic acid or esters thereof.

If an α-keto acid ester is produced by the method of the invention, said ester may be any type of organic ester. Preferred esters are methyl esters, ethyl esters, isopropyl esters, tert-butyl esters and benzyl esters, most preferred esters are methyl esters.

α-Keto acids or esters thereof obtained by the method of the invention may be further transformed into derivates, e.g. converted to α-keto amides or to salts of α-keto acids by methods known in the art. Preferably, the method is used to produce pyruvic acid, more preferably ¹³C₁-pyruvic acid, and the ¹³C₁-pyruvic acid obtained is converted into a salt, i.e. a ¹³C₁-pyruvate. This is preferably done by using a work-up procedure in step c) of the method of the invention by which the salt, i.e. ¹³C₁-pyruvate is obtained.

Preferred salts are those ¹³C₁-pyruvates which comprise an inorganic cation from the group consisting of NH₄⁺, K⁺, Rb⁺, Cs⁺, Ca²⁺, Sr²⁺ and Ba²⁺, preferably NH₄⁺ , K⁺, Rb⁺ or Cs⁺, more preferably K⁺, Rb⁺, Cs⁺ and most preferably Cs⁺, as in detail described in the International Patent Application No. PCT/NO07/00109. Such salts are preferably used for the production of hyperpolarised ¹³C₁-pyruvate by the DNP method. The aforementioned salts are preferably obtained by using a work-up procedure in step c) of the method of the invention by which these salts are obtained.

Further preferred are ¹³C₁-pyruvates of an organic amine or amino compound, preferably TRIS-¹³C₁-pyruvate or meglumine-¹³C₁-pyruvate, as in detail described in International Patent Application No. PCT/NO06/00481. Again such salts are preferably used for the production of hyperpolarised ¹³C₁-pyruvate by the DNP method. The aforementioned salts are preferably obtained by producing ¹³C-pyruvic acid according the method of the invention and converting said ¹³C-pyruvic acid to the aforementioned salts.

In step a) of the method of the invention, a compound of formula (II) 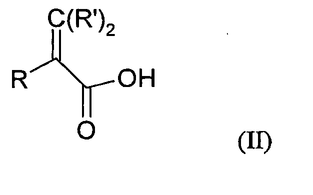 wherein
- R: is a straight chain or branched C₁-C₆-alkyl group or phenyl or benzyl, optionally substituted with one or more hydroxyl or carboxyl groups; and
- R': is the same or different and denotes H or straight chain or branched C₁-C₃- alkyl
is obtained by carboxylation of a compound of formula (III) 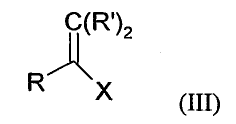 wherein R and R' are as defined above and X is Br, Cl or I.

If compounds of formula (III) are used in step a), wherein R contains hydroxyl or carboxyl groups, said groups are preferably protected, e.g. by suitable protection groups known in the art. For instance, hydroxyl groups may be protected as silyl ethers, derived from the reaction of the free hydroxyl group with compounds like tert-butyldimethylsilyl chloride or trimethylsilyl chloride. Carboxyl groups may be protected as oxazolines or ortho-esters. The former may be produced by reaction of the free carboxyl group or the acid chloride thereof with 2-amino-2-methyl-1-propanol and subsequent reaction with thionyl chloride. The latter may be synthesized by reaction of the acid chloride of with 3-methyl-3-hydroxymethyloxethan and subsequent reaction with boron trifluoro ethylether.

Compounds of formula (III) are commercially available compounds and several methods known in the art may be used to carry out the carboxylation of compounds of formula (III) to obtain compounds of formula (II). In one embodiment, catalytic insertion of CO results in compounds of formula (II). Catalytic insertion of CO might be carried out in the following way: compounds of formula (III), preferably compounds of formula (III) wherein X is Br or I, are dissolved in a suitable solvent and portion wise added to a solution of sodium hydroxide and a catalyst, e.g. nickel cyanide and cetyltrimethylammonium bromide in a suitable solvent under CO-atmosphere. The method is for instance described in H. Alper et al., Tetrahedron Lett. Vol. 30, (20), 1989, 2615-2616. The crude reaction product may or may not be purified before step b) or c) of the method according to the invention. If the crude reaction product is purified, said crude reaction product is preferably distilled or crystallised. To obtain compounds of formula (II) which are ¹³C₁-enriched - such ¹³C₁-enriched compounds of formula (II) are needed as intermediates if the method of the invention is used to produce ¹³C₁-enriched α-keto acids or esters thereof - ¹³CO, which is a commercially available compound, is used in the procedure as described above.

In a more preferred embodiment, carboxylation is carried out by lithiation of compounds of formula (III) and subsequent reaction with CO₂. Briefly, compounds of formula (III), preferably compounds of formula (III) wherein X is Br or I, are dissolved in an aprotic polar solvent like tetrahydrofuran (THF) and cooled to a temperature below 0 °C. An organolithium reagent like for instance t-BuLi in a suitable solvent is added and the mixture is stirred to generate a lithio-derivative of compound of formula (III), e.g. a compound of formula (III) wherein X is Li. The method is described in for instance C. Wang et al., J. Org. Chem 2005, 70, 5150-5156 or D. Jeffery et al., Org. Lett 7, (8), 2005, 1581-1584. The reaction mixture is then reacted with CO₂ and release of the carboxyl function, e.g. by extraction with suitable organic solvents like dichloromethane or by distillation, yields the compounds of formula (II). The crude reaction product may or may not be purified before step b) or c) of the method according to the invention. If the crude reaction product is purified, said crude reaction product is preferably distilled or crystallised. To obtain ¹³C₁-enriched compounds of formula (II), ¹³CO₂, which is a commercially available compound, is used in the procedure as described above. Any type of CO₂ may be used in the reaction. If the reaction is carried out on a low scale, solid CO₂ may be used. However, for large scale reactions and for reactions wherein ¹³CO₂ is used, it is preferred to use gaseous CO₂ or supercritical CO₂, since it is possible to re-circulate non-used CO₂, especially in a continuous process.

In a most preferred embodiment, carboxylation is carried out by a Grignard reaction. Briefly, a Grignard reagent is generated from a compound of formula (III), preferably a compound of formula (III) wherein X is Br, and magnesium in a suitable solvent like THF and the Grignard reagent is then reacted with CO₂. Release of the carboxyl function as described in the preceding paragraph yields the compounds of formula (II). A similar reaction is for instance described in M. Matarrese et al., Applied Radiation and Isotopes 57, 2002, 675-679. The crude reaction product may or may not be purified before step b) or c) of the method according to the invention. If the crude reaction product is purified, said crude reaction product is preferably distilled or crystallised. To obtain ¹³C₁-enriched compounds of formula (II), ¹³CO₂, which is a commercially available compound, is used in the procedure as described above. Any type of CO₂ may be used in the reaction. If the reaction is carried out on a low scale, solid CO₂ may be used. However, for large scale reactions and for reactions wherein ¹³CO₂ is used, it is preferred to use gaseous CO₂ or supercritical CO₂, since it is possible to re-circulate non-used CO₂, especially in a continuous process.

In a preferred embodiment of the reaction described in the previous paragraph, said reaction is carried out in a micro reactor, e.g. a microchannel reaction system type micro reactor, wherein the microchannel is coated with a thin film of the Grignard reagent, or wherein the Grignard reagent is immobilized inside a column packed with a suitable inert material to adsorb the Grignard reagent. The micro reactor is preferably flushed with an inert gas like argon or nitrogen before carbon dioxide gas is flushed through. With this method, excess carbon dioxide may be recovered downstream and re-used, which is of course especially important if ¹³CO₂ is used. The carboxyl function may be released and the compound of formula (II) may be recovered from the micro reactor by flushing the reactor with for instance a basic aqueous solution (with or without addition of an organic solvent). Alternatively, the ester of a compound of formula (II) may be obtained by flushing the reactor with an alcohol, for instance ethanol.

In another preferred embodiment the reaction described above is carried out in a continuous micro reactor system where a solution of Grignard reagent is mixed with a CO₂ flow inside a continuous micro reactor system. By using monitoring techniques and tools like for instance Process Analytical Tool (PAT) a steady state condition can be obtained at the end of the reactor, for instance by regulating the flow of CO₂ into the reactor for tuning the consumptions of reactants and thus ensuring the most possible efficient use of said reactants.

In step b) of the method of the invention, the compounds of formula (II) obtained from step a) are optionally converted into esters. The conversion of carboxylic acids into esters, the so-called esterification, is a method well known in the art wherein a carboxylic acid is reacted with an alcohol in the presence of protons. The crude reaction product may be used in step c). In a preferred embodiment, the methyl esters of the compounds of formula (II) are prepared by reaction of the compounds of formula (II) with methanol.

In step c) of the method of the invention the compounds of formula (II) or esters thereof are oxidised to obtain the α-keto acids of formula (I) or esters thereof. Several methods known in the art are suitable to oxidise the compounds of formula (II) or esters thereof.

Suitably, compounds of formula (II) in the form of their esters can be oxidised with hydrogen peroxide in the presence of chromium catalysts. Briefly, CrO₃, trimethylamine, hydrogen peroxide and the compound of formula (II) in the form of its ester are added in acetonitrile and heated to obtain an α-keto acid ester of formula (I). The method is for instance described in M. Inoue et al., Chemistry Letters 1989, 99-100.

Alternatively, compounds of formula (II) in the form of their esters can be oxidised using molecular oxygen and a ruthenium catalyst. Briefly, oxygen is bubbled through a mixture of RuO₂, acetone, pyromellitic acid and acetaldehyde, and a solution of a compound of formula (II) in the form of its ester in acetone is added. The mixture is stirred under oxygen atmosphere and quenched with sodium carbonate to obtain an α-keto acid of formula (I). The method is described in K. Kaneda et al, Chem. Commun. 1990, 1467-1468.

In a preferred embodiment, compounds of formula (II) or esters thereof are oxidised by ozonolysis. Briefly, ozonolysis is carried out in a suitable solvent like for instance dichloromethane or methanol at moderate to low temperatures. In a more preferred embodiment, compounds of formula (II) are used as starting material for the ozonolysis. Ozonolysis is described in for instance Y. Hon et al., Tetrahedron 60 (2004), 4837-4860, in T. Chan et al., J. Org. Chem. 60, 1995, 4228-4232 or in S.G. van Ornum et al., Chem. Rev. 2006, 2990-3001.

The ozonolysis reaction may be carried out in a micro reactor system / continuous micro reactor system as described for the Grignard reaction on page 9.

If the reaction product of step c) is an ester of an α-keto acid of formula (I), e.g. because the carboxyl group had to be protected in the oxidation of step c), the free α-keto acid of formula (I) can be obtained by methods well known in the art, i.e. by acid- or base catalyzed hydrolysis.

If R in formula (I) contains hydroxyl and/or carboxyl groups which were protected in the previous reaction steps, these protection groups are removed after step c) or optional step d). Briefly, hydroxyl groups protected as silyl ethers may be released by reaction with fluoride reagents like tetrabutylammonium fluoride or hydrogen fluoride. Carboxyl groups protected as oxazolines or ortho-esters may be released by reaction with acids like hydrogen chloride.

In a preferred embodiment, the invention provides a method for producing pyruvic acid or esters thereof, said method comprising:
a) obtaining a compound of formula (II) 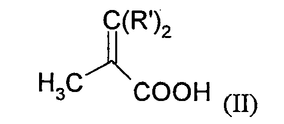 wherein R' is the same or different and denotes H or straight chain or branched C₁-C₃-alkyl by carboxylation of a compound of formula (III) 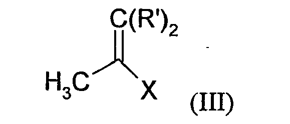 wherein R' is defined as above and X is selected from Cl, Br or I
b) optionally converting the compound of formula (II) into an ester;
c) oxidising the compound of formula (II) or the ester thereof to obtain pyruvic acid or the ester thereof; and
d) if an ester of pyruvic acid was obtained in step c), optionally converting the ester into pyruvic acid.

In a preferred embodiment, R' is the same and denotes H. In another preferred embodiment, R' is different and denotes H and methyl.

In a more preferred embodiment, the compound of formula (II) of step a) is obtained by lithiation of the compound of formula (III) with organolithium reagents, preferably organolithium reagents like n-BuLi, t-BuLi or sec-BuLi/TMEDA. The lithiation reaction is carried out in suitable solvents, e.g. aprotic polar solvent like tetrahydrofuran (THF) at low temperatures e.g. temperatures of -40 °C and below. The product of the lithiation reaction is then reacted with CO₂ and release of the carboxyl function, e.g. by extraction with suitable organic solvents like dichloromethane or by distillation, yields the compounds of formula (II). Any type of CO₂ may be used in the reaction, i.e. solid CO₂, gaseous CO₂ or supercritical CO₂. The crude reaction product may or may not be purified before step b) or c) of the method according to the invention. If the crude reaction product is purified, said crude reaction product is preferably distilled.

The reaction is shown in reaction scheme 1:

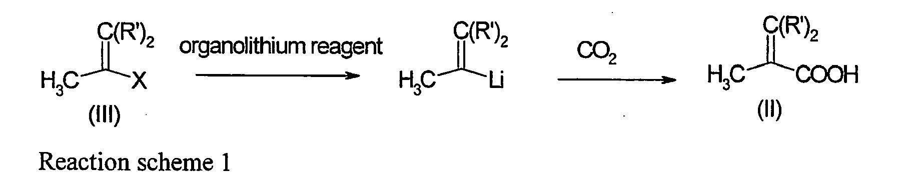

In a most preferred embodiment, the compound of formula (II) of step a) is obtained by reaction of a compound of formula (III), preferably a compound of formula (II) wherein X is Br with magnesium to form the Grignard reagent. The reaction is suitably carried out at room temperature or above in a polar aprotic solvent like THF. The Grignard reagent is then reacted with CO₂ and release of the carboxyl function, e.g. by extraction with suitable organic solvents like dichloromethane or by distillation, yields the compounds of formula (II). Again any type of CO₂ may be used in the reaction. The crude reaction product may or may not be purified before step b) or c) of the method according to the invention. If the crude reaction product is purified, said crude reaction product is preferably distilled.

The reaction is shown in reaction scheme 2:

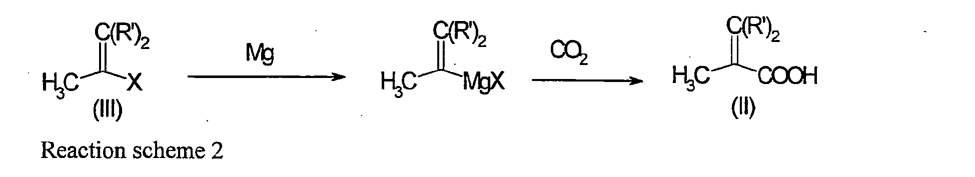

The conversion of the compound of formula (II) into to an ester in step b) might or might not be carried out, depending on the method of oxidation which is chosen for step c). In a preferred embodiment, the oxidation method is ozonolysis and if pyruvic acid itself is the desired reaction product, conversion of the compound of formula (II) into an ester in step b) is preferably not carried out.

If the method described above is used to produce an ester of pyruvic acid, the conversion of the compound of formula (II) into an ester is conveniently carried out in step b) by methods well known in the art. Preferably, the methyl ester, by reaction of the compound of formula (II) with methanol and said crude ester is used in step c) of the method described above without any further purification steps.

In a preferred embodiment of the method above, the compound of formula (II) or the ester thereof, if step b) has been carried out, is oxidised to pyruvic acid or the ester thereof by ozonolysis, preferably by ozonolysis in dichloromethane, methanol or a mixture thereof as a solvent at moderate to low temperatures, i.e. room temperature to temperatures of about 0 °C and below, e.g. -78 °C. The completion of the reaction is indicated by a blue colour in the reaction mixture caused by an excess of free ozone. Said free ozone may be removed by passing a stream of for instance nitrogen gas or oxygen gas through the reaction mixture. An (unstable) ozonoid is formed during the course of the reaction. If the reaction is carried out at moderate temperatures said ozonoid will react to the desired pyruvic acid or ester thereof. At low temperatures an oxidizing or reducing agent is needed to convert the ozonoid to the desired reaction product. Oxidizing agents are known in the art and examples of suitable oxidizing agents are peroxyacids, silver oxide, chromic acid, oxygen, permanganates or hydrogen peroxide. Reducing agents are known in the art and examples of suitable reducing agents are zinc acetic acid, sulfite ions, bisulfite ions, iodide, dimethylsulfide or thiourea.

In one embodiment, the solvent is evaporated and the crude reaction product is preferably purified, preferably by distillation. In another embodiment, the solvent is evaporated and the crude reaction product is purified by crystallization. This may be done by evaporating the solvent and addition of water and a base to convert pyruvic acid into a salt, i.e. a pyruvate. The mixture is then left for crystallization. In a preferred embodiment, said base in an inorganic sodium base, preferably NaOH.

Obtaining the salt rather than the free pyruvic acid is of advantage with regard to shelf life/stability. Pyruvic acid is not a very stable compound and by storing pyruvic acid in the form of its salts, e.g. in the form of sodium pyruvate this problem can be overcome. Alternatively, the pyruvate obtained by crystallization may be converted into free pyruvic acid in a subsequent step and method for this conversion are known in the art.

The ozonolysis reaction is shown in reaction scheme 3:

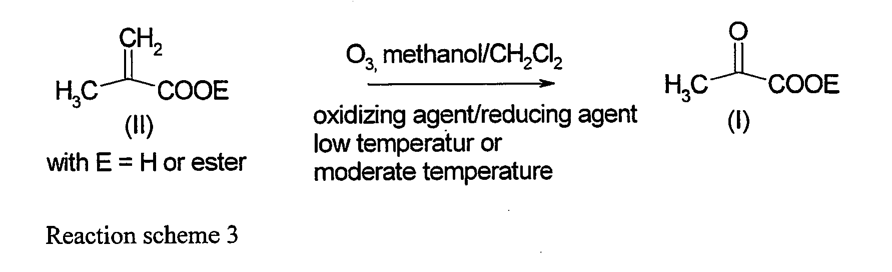

If an ester of pyruvic acid was obtained in step c) said ester may optionally be converted into pyruvic acid by methods well known in the art, e.g. acid- or base catalyzed hydrolysis.

In a more preferred embodiment, the invention provides a method for producing ¹³C₁-pyruvic acid or esters thereof, said method comprising:
a) obtaining a compound of formula (II) 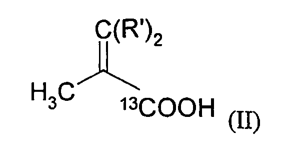 wherein R' is the same or different and denotes H or straight chain or branched C₁-C₃-alkyl by carboxylation of a compound of formula (III) 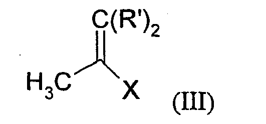 wherein R' is defined as above and X is selected from Cl, Br or I
b) optionally converting the compound of formula (II) into an ester;
c) oxidising the compound of formula (II) or the ester thereof to obtain ¹³C₁-pyruvic acid or the ester thereof; and
d) if an ester of ¹³C₁-pyruvic acid was obtained in step c), optionally converting the ester into ¹³C₁-pyruvic acid.

In a preferred embodiment, R' is the same and denotes H. In another preferred embodiment, R' is different and denotes H and methyl.

The method of the invention for producing ¹³C₁-pyruvic acid or esters thereof is carried out as described for the method of the invention for producing pyruvic acid or esters thereof, however, instead of CO₂, ¹³CO₂ is used in reaction step a). Further it is preferred to purify the crude reaction product of step a) by distillation and the final reaction product of step c) or d) by distillation. In another embodiment, the final reaction product of step c) or d) is purified by crystallization as described above by converting pyruvic acid into a salt, i.e. a pyruvate.

In a preferred embodiment, the method of the invention is used for producing ¹³C₁-pyruvic acid, i.e. step d) is not carried out and the reaction product of step c) is purified by distillation.

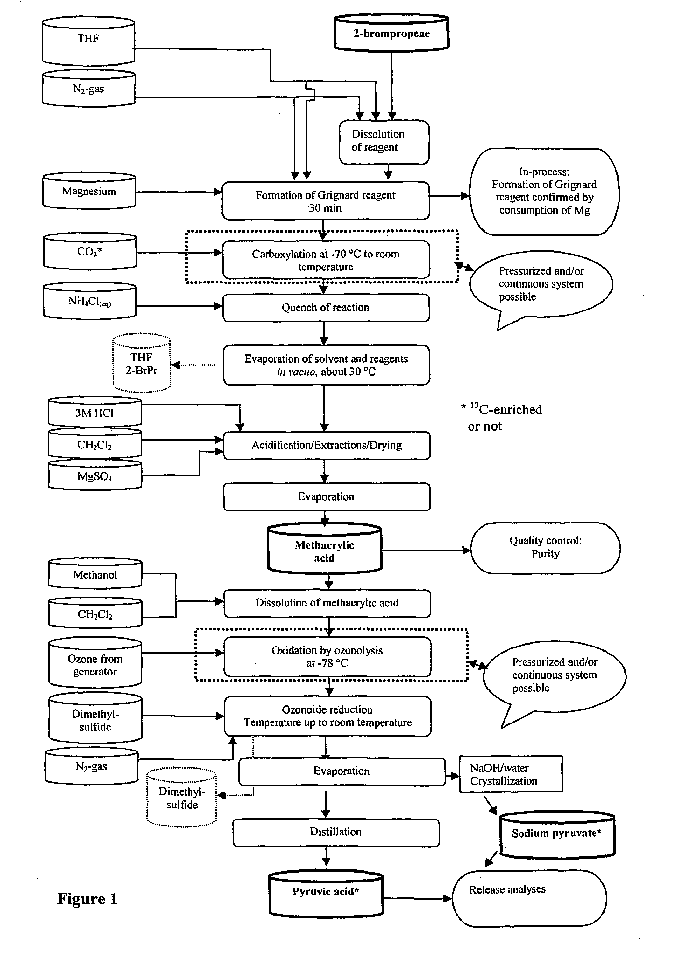

In another preferred embodiment, the method invention is used for producing ¹³C₁-pyruvate, more preferably sodium ¹³C₁-pyruvate, i.e. step d) is not carried out and the reaction product of step c) is purified by crystallization as described above. Figure 1 shows a flow chart of a preferred embodiment of the invention for the production of pyruvic acid or ¹³C₁-pyruvic acid in the form of the free acid or in the form of its sodium salt.

The invention is further illustrated by way of the Examples.

### Examples

### Example 1a Synthesis of methacrylic acid, a compound of formula (II), by Grignard reaction starting from 2-bromopropene, a compound of formula (III)

2-Bromopropene (24.20 g, 200 mmol) dissolved in tetrahydrofuran (THF, 100 ml) was added drop wise to a stirred suspension of magnesium turnings (4.38 g, 180 mmol) in THF (100 ml) under nitrogen-atmosphere. The 2-bromopropene was added at a rate causing the reaction mixture to boil gently. After complete formation of the Grignard reagent had been achieved, the mixture was stirred at room temperature for 30 min, before being cooled to - 70 °C. The reaction mixture was subjected to an atmosphere of carbon dioxide (1 bar) under heavy stirring (vortexing). The reaction was allowed to reach room temperature and then terminated by addition of saturated aqueous ammonium chloride (100 ml). THF and excess 2-bromopropene was removed *in vacuo* at 30 °C and the aqueous phase was acidified by addition of aqueous HCl (3M, 50 ml) before extraction with dichloromethane (3 x 50 ml). The combined organic extracts were dried over magnesium sulfate and evaporated to give the title compound; a colourless or slightly yellow oil. Yield: 13.50 g (87 %), purity 99%.

### Example 1b Synthesis of methacrylic acid, a compound of formula (II), by catalytic insertion of CO starting from 2-bromopropene, a compound of formula (III)

To a mixture of tetrakis(triphenylphosphine)palladium (0.29 g, 0.25 mmol), hexadecyltrimethylammonium bromide (0.36 g, 1.0 mmol) and 2-bromopropene (1.16 g, 10 mmol) in toluene (20 ml) was added aqueous NaOH (5M, 20 ml, 100 mmol). The mixture was subjected to an atmosphere of carbon monoxide (1 bar) and heated to 95 °C for 2 h. The phases were separated and the organic phase was extracted with aqueous NaOH (1 M, 2 x 20 ml). The aqueous phase was washed with dichloromethane (50 ml). The aqueous phase was acidified using aqueous HCl (3 M) and extracted with dichloromethane (3 x 50 ml). The combined organic extracts were dried over magnesium sulfate and evaporated *in vacuo* at 30 °C to give the product; a colourless or slightly yellow oil. Yield: 0.52 g (60 %).

### Example 2 Synthesis of pyruvic acid, a compound of formula (I) by ozonolysis of methacrylic acid, a compound of formula (II)

A solution of methacrylic acid obtained in Example 1a or 1b (8.61 g, 100 mmol) was dissolved in dichloromethane/methanol (1:1 v/v, 50 ml) and cooled to -78 °C. A stream of ozone was passed through the solution until the solution turned blue. Dimethylsulfide (12.41 g, 200 mmol) was added and the mixture was allowed to reach room temperature. The reaction mixture was evaporated *in vacuo* and the residue was distilled. The product was isolated as colourless oil. Yield: 7.95 g (90 %).

### Example 3 Synthesis of pyruvic acid, a compound of formula (I) by ozonolysis of methacrylic acid, a compound of formula (II)

A solution of methacrylic acid obtained in Example 1a or 1b (4.68 g, 54 mmol) in dichloromethane/methanol (5 % methanol, 50 ml) was cooled to -78 °C and a stream of ozone was passed through until the solution turned blue. Dimethylsulfide (12.41 g, 200 mmol) was added and the mixture was allowed to reach room temperature. Excess dimethylsulfide was removed by passing a stream of nitrogen through the reaction mixture. The reaction mixture was evaporated *in vacuo* at 30 °C and the residue was distilled. The product was isolated as colourless oil. Yield: 4.40 g (98 %). Purity: 97 %.

### Example 4 Synthesis of pyruvate, a salt of a compound of formula (II) by ozonolysis of methacrylic acid, a compound of formula (II)

A solution of methacrylic acid obtained in Example 1a or 1b (15.31 g, 178 mmol) in dichloromethane/methanol (5 % methanol, 50 ml) was cooled to -78 °C and a stream of ozone was passed through until the solution turned blue. Dimethylsulfide (12.41 g, 200 mmol) was added and the mixture was allowed to reach room temperature. Excess dimethylsulfide was removed by passing a stream of nitrogen through the reaction mixture. The reaction mixture was evaporated *in vacuo* at 30 °C. To the residue was added water (100 ml) and then slowly added a solution of aq. NaOH (1M, 178 ml). The mixture was concentrated and left for crystallisation at 4 °C. The white crystalline material obtained was filtered and washed with acetone (3x100 ml). Yield: 14.50 g (92 %). Purity: 95 %.

## Claims

1. Method for producing an α-keto acid of formula (I) or an ester thereof 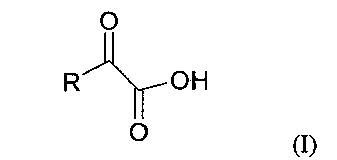 wherein R is a straight chain or branched C₁-C₆-alkyl group or phenyl or benzyl, optionally substituted with one or more hydroxyl or carboxyl groups, said method comprising:
a) obtaining a compound of formula (II) 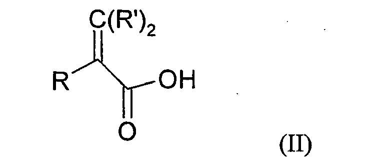
wherein R is defined as above and R' is the same or different and denotes H or a straight chain or branched C₁-C₃-alkyl group, by carboxylation of a compound of formula (III) 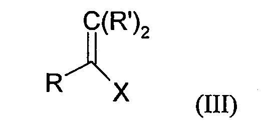 wherein R and R' are as defined above and X is Cl, Br or I;
b) optionally converting the compound of formula (II) into an ester;
c) oxidising the compound of formula (II) or the ester thereof to obtain an α-keto acid of formula (I) or an ester thereof; and
d) if an ester of the α-keto acid of formula (I) was obtained in step c), optionally converting the ester into a α-keto acid.

2. Method according to claim 1 wherein R' is the same and denotes H or wherein R' is different and denotes H and methyl.

3. Method according to claims 1 and 2 wherein R is methyl, ethyl, n-propyl, isopropyl, n-butyl, isobutyl, tert-butyl, phenyl, benzyl, CH₂-COOH, CH₂-CH₂-COOH, CH₂-(CH₂)₂-COOH, CH₂-(CH₂)₃-COOH or CHOH-(CHOH)₂-CH₂OH.

4. Method according to claims 1 to 3 wherein carboxylation in step a) is carried out by lithiation of the compound of formula (III) with an organolithium reagent and subsequent reaction with CO₂.

5. Method according to claims 1 to 3 wherein carboxylation in step a) is carried out by generating a Grignard reagent from the compound of formula (III) and magnesium and subsequent reaction of said Grignard reagent with CO₂.

6. Method according to claims 1 to 5 wherein the oxidation in step c) is carried out by ozonolysis.

7. Method according to claim 1 for the production of pyruvic acid or an ester thereof, said method comprising:
a) obtaining a compound of formula (II) 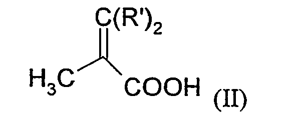 wherein R' is the same or different and denotes H or a straight chain or branched C₁-C₃-alkyl group by carboxylation of a compound of formula (III) 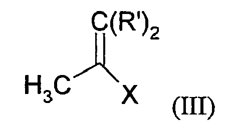 wherein R' is defined as above and X is selected from Cl, Br or I
b) optionally converting the compound of formula (II) into an ester;
c) oxidising the compound of formula (II) or the ester thereof to obtain pyruvic acid or the ester thereof; and
d) if an ester of pyruvic acid was obtained in step c), optionally converting the ester into pyruvic acid.

8. Method according to claim 7 wherein carboxylation in step a) is carried out by lithiation of the compound of formula (III) with an organolithium reagent and subsequent reaction with CO₂.

9. Method according to claim 7 wherein carboxylation in step a) is carried out by generating a Grignard reagent from the compound of formula (III) and magnesium and subsequent reaction of said Grignard reagent with CO₂.

10. Method according to claims 7 to 9 wherein the oxidation in step c) is carried out by ozonolysis.

11. Method according to claims 1 to 6 wherein the α-keto acid of formula (I) or the ester thereof produced by the method is an ¹³C₁-α-keto acid or an ester thereof.

12. Method according to claims 7 to 10 wherein the pyruvic acid or ester thereof produced by the method is ¹³C₁-pyruvic acid or an ester thereof.

13. Method according to claims 10 and 11 wherein carboxylation in step a) is carried out by lithiation of the compound of formula (III) with an organolithium reagent and subsequent reaction with ¹³CO₂.

14. Method according to claim 10 and 11 wherein carboxylation in step a) is carried out by generating a Grignard reagent from the compound of formula (III) and magnesium and subsequent reaction of said Grignard reagent with ¹³CO₂.

## Patentansprüche

1. Verfahren zur Herstellung einer α-Ketosäure nach Formel (I) oder eines Esters
davon, 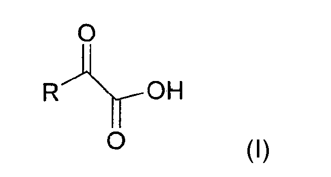 wobei R eine geradkettige oder verzweigte C₁-C₆-Alkyl-, Phenyl-, oder Benzylgruppe ist, wahlweise substituiert mit einer oder mehreren Hydroxyl- oder Carboxylgruppen, das Verfahren umfassend:
a) Gewinnung einer Verbindung der Formel (II), 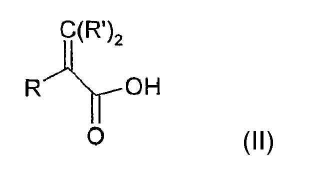
wobei R wie oben definiert und R' gleich oder verschieden ist und H oder eine geradkettige oder verzweigte C₁-C₃-Alkylgruppe bezeichnet, durch Carboxylierung einer Verbindung nach Formel (III), 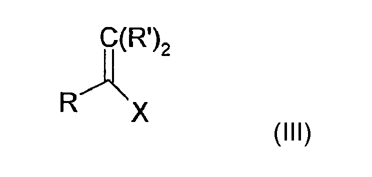 wobei R und R' wie oben definiert sind und X Cl, Br oder I ist;
b) wahlweises Umwandeln der Verbindung nach Formel (II) in einen Ester;
c) Oxidieren der Verbindung nach Formel (II) oder des Esters davon, um eine α-Ketosäure nach Formel (I) oder einen Ester davon zu erhalten; und
d) falls ein Ester der α-Ketosäure nach Formel (I) in Schritt c) erhalten wurde, wahlweise Umwandlung des Esters in eine α-Ketosäure.

2. Verfahren nach Anspruch 1, wobei R' gleich ist und H bezeichnet oder wobei R' verschieden ist und H und Methyl bezeichnet.

3. Verfahren nach Anspruch 1 oder 2, wobei R Methyl, Ethyl, n-Propyl, Isopropyl, n-Butyl, Isobutyl, tert-Butyl, Phenyl, Benzyl, CH₂-COOH, CH₂-CH₂-COOH, CH₂-(CH₂)₂-COOH, CH₂-(CH₂)₃-COOH oder CHOH-(CHOH)₂-CH₂OH ist.

4. Verfahren nach einem der Ansprüche 1 bis 3, wobei die Carboxylierung in Schritt a) durch Lithiierung der Verbindung nach Formel (III) mit einem Organolithiumreagenz und anschließender Reaktion mit CO₂ durchgeführt wird.

5. Verfahren nach einem der Ansprüche 1 bis 3, wobei die Carboxylierung in Schritt a) durch Bildung eines Grignard-Reagenzes aus der Verbindung nach Formel (III) und Magnesium und anschließender Reaktion des Grignard-Reagenzes mit CO₂ durchgeführt.

6. Verfahren nach einem der Ansprüche 1 bis 5, wobei die Oxidierung in Schritt c) durch Ozonolyse durchgeführt wird.

7. Verfahren nach Anspruch 1 zur Herstellung von Brenztraubensäure oder eines Esters davon, das Verfahren umfassend:
a) Gewinnung einer Verbindung der Formel (II), 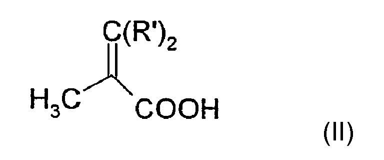 wobei R' gleich oder verschieden ist und H oder eine geradkettige oder verzweigte C₁-C₃-Alkylgruppe bezeichnet, durch Carboxylierung einer Verbindung nach Formel (III), 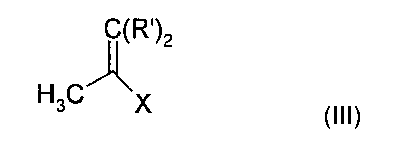 wobei R' wie oben definiert ist und X ausgewählt ist aus Cl, Br oder I;
b) wahlweises Umwandeln der Verbindung nach Formel (II) in einen Ester;
c) Oxidieren der Verbindung nach Formel (II) oder des Esters davon, um Brenztraubensäure oder einen Ester davon zu erhalten; und,
d) falls ein Ester von Brenztraubensäure in Schritt c) erhalten wurde, wahlweise Umwandlung des Esters in Brenztraubensäure.

8. Verfahren nach Anspruch 7, wobei die Carboxylierung in Schritt a) durch Lithiierung der Verbindung nach Formel (III) mit einem Organolithiumreagenz und anschließender Reaktion mit CO₂ durchgeführt wird.

9. Verfahren nach Anspruch 7, wobei die Carboxylierung in Schritt a) durch Bildung eines Grignard-Reagenzes aus der Verbindung nach Formel (III) und Magnesium und anschließender Reaktion des Grignard-Reagenzes mit CO₂ durchgeführt wird.

10. Verfahren nach einem der Ansprüche 7 bis 9, wobei die Oxidierung in Schritt c) durch Ozonolyse durchgeführt wird.

11. Verfahren nach einem der Ansprüche 1 bis 6, wobei die nach dem Verfahren hergestellte α-Ketosäure nach Formel (I) oder des Esters davon eine ¹³C₁-α-Ketosäure oder ein Ester davon ist.

12. Verfahren nach einem der Ansprüche 7 bis 10, wobei die nach dem Verfahren hergestellte Brenztraubensäure oder des Esters davon eine ¹³C₁-Brenztraubensäure oder ein Ester davon ist.

13. Verfahren nach Anspruch 10 oder 11, wobei die Carboxylierung in Schritt a) durch Lithiierung der Verbindung nach Formel (III) mit einem Organolithiumreagenz und anschließender Reaktion mit ¹³CO₂ durchgeführt wird.

14. Verfahren nach Anspruch 10 oder 11, wobei die Carboxylierung in Schritt a) durch Bildung eines Grignard-Reagenzes aus der Verbindung nach Formel (III) und Magnesium und anschließender Reaktion des Grignard-Reagenzes mit ¹³CO₂ durchgeführt wird.

## Revendications

1. Procédé de production d'un acide α-céto de formule (I) ou d'un ester de celui-ci 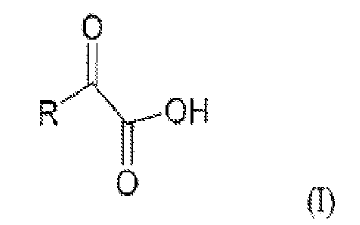 dans laquelle R est un groupe alkyle en C₁ à C₆ à chaîne linéaire ou ramifiée ou phényle ou benzyle, éventuellement substitué par un ou plusieurs groupes hydroxyles ou carboxyles, ledit procédé comprenant :
a) l'obtention d'un composé de formule (II) 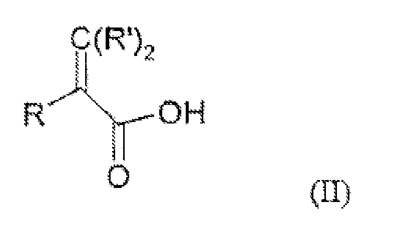
dans laquelle R est défini comme précédemment et R' est identique ou différent et désigne H ou un groupe alkyle en C₁ à C₃ à chaîne linéaire ou ramifiée, par carboxylation d'un composé de formule (III) 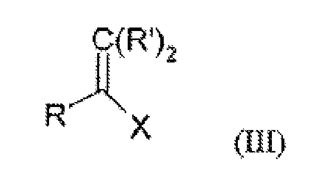 dans laquelle R et R' sont tels que définis précédemment et X représente CI, Br ou I ;
b) en variante, la conversion du composé de formule (II) en un ester ;
c) l'oxydation du composé de formule (II) ou de son ester afin d'obtenir un acide α-céto de formule (I) ou un ester de celui-ci ; et
d) en variante, si un ester de l'acide α-céto de formule (I) est obtenu à l'étape c), la conversion de l'ester en acide α-céto.

2. Procédé selon la revendication 1, dans lequel R' est identique et désigne H ou dans lequel R' est différent et désigne H et un méthyle.

3. Procédé selon les revendications 1 et 2, dans lequel R est du méthyle, de l'éthyle, du n-propyle, de l'isopropyle, du n-butyle, de l'isobutyle, du tert-butyle, du phényle, du benzyle, du CH₂-COOH, du CH₂-CH₂-COOH, du CH₂-(CH₂)₂-COOH, du CH₂-(CH₂)₃-COOH ou du CHOH-(CHOH)₂-CH₂OH.

4. Procédé selon les revendications 1 à 3, dans lequel la carboxylation à l'étape
a) est mise en oeuvre par lithiation du composé de formule (III) avec un réactif organolithium et par réaction consécutive avec du CO₂.

5. Procédé selon les revendications 1 à 3, dans lequel la carboxylation à l'étape
a) est mise en oeuvre par production d'un réactif de Grignard à partir du composé de formule (III) et de magnésium et par réaction consécutive dudit réactif de Grignard avec du CO₂.

6. Procédé selon les revendications 1 à 5, dans lequel l'oxydation à l'étape c) est mise en oeuvre par ozonolyse.

7. Procédé selon la revendication 1, afin d'assurer la production d'acide pyruvique ou d'un ester de ce dernier, ledit procédé comprenant :
a) l'obtention d'un composé de formule (II) 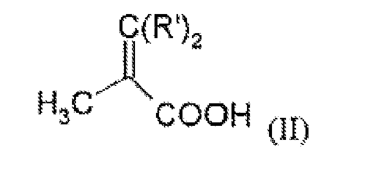 dans laquelle R' est identique ou différent et désigne H ou un groupe alkyle en C₁ à C₃ à chaîne linéaire ou ramifiée, par carboxylation d'un composé de formule (III) 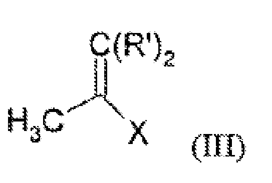 dans laquelle R' est défini comme précédemment et X est sélectionné à partir de CI, Br ou I
b) en variante, la conversion du composé de formule (II) en un ester ;
c) l'oxydation du composé de formule (II) ou de son ester afin d'obtenir de l'acide pyruvique ou un ester de ce dernier ; et
d) en variante, si un ester d'acide pyruvique est obtenu à l'étape c), la conversion de l'ester en acide pyruvique.

8. Procédé selon la revendication 7, dans lequel la carboxylation à l'étape a) est mise en oeuvre par lithiation du composé de formule (III) avec un réactif organolithium et par réaction consécutive avec du CO₂.

9. Procédé selon la revendication 7, dans lequel la carboxylation à l'étape a) est mise en oeuvre par production d'un réactif de Grignard à partir du composé de formule (III) et de magnésium et par réaction consécutive dudit réactif de Grignard avec du CO₂.

10. Procédé selon les revendications 7 à 9, dans lequel l'oxydation à l'étape c) est mise en oeuvre par ozonolyse.

11. Procédé selon les revendications 1 à 6, dans lequel l'acide α-céto de formule (I) ou l'ester de celui-ci produit par le procédé est un acide ¹³C₁-α-céto ou un ester de celui-ci.

12. Procédé selon les revendications 7 à 10, dans lequel l'acide pyruvique ou son ester produit par le procédé est de l'acide ¹³C₁-pyruvique ou un ester de celui-ci.

13. Procédé selon les revendications 10 et 11, dans lequel la carboxylation à l'étape a) est mise en oeuvre par lithiation du composé de formule (III) avec un réactif organolithium et par réaction ultérieure avec du ¹³CO₂.

14. Procédé selon les revendications 10 et 11 dans lequel la carboxylation à l'étape a) est mise en oeuvre par production d'un réactif de Grignard à partir du composé de formule (III) et de magnésium et par réaction consécutive dudit réactif de Grignard avec du ¹³CO₂.
